## Europäisches Patentamt
## European Patent Office
## Office européen des brevets

(11) Publication number: **0 135 328**
**B1**

# EUROPEAN PATENT SPECIFICATION

(45) Date of publication of the patent specification: 25.02.87

(51) Int. Cl.⁴: **C 07 D 213/807**

(21) Application number: **84305273.9**

(22) Date of filing: **03.08.84**

(54) A process for the manufacture of quinolinic acid.

(30) Priority: **08.06.83 US 521211**

(43) Date of publication of application:
**27.03.85 Bulletin 85/13**

(45) Publication of the grant of the patent:
**25.02.87 Bulletin 87/9**

(84) Designated Contracting States:
**AT BE CH DE FR GB IT LI NL SE**

(56) References cited:
**EP-A-0 024 197**
**EP-A-0 034 943**

**CHEMICAL ABSTRACTS, vol. 83, no. 3, July 21, 1975, Columbus, Ohio, USA, D.C. AYRES et al. "Action of ruthenium tetraoxide on derivatives of naphthalene and its monoaza analogs", page 485, column 2, abstract no. 27 980v**
**CHEMICAL ABSTRACTS, vol. 57, no. 12, December 10, 1962, Columbus, Ohio, USA, A. CORBELLINI "Catalytic oxidation of nitrogen containing heterocyclics", column 15080c**

(73) Proprietor: **APPLETON PAPERS INC., P.O. Box 359 825 East Wisconsin Avenue, Appleton Wisconsin 54912 (US)**

(72) Inventor: **Mathiaparanam, Ponnampalam, 2314 S. Gladys Avenue, Appleton Wisconsin 54915 (US)**

(74) Representative: **Roberts, Jonathan Winstanley, The Wiggins Teape Group Limited Group Patents Dept. Butler's Court, Beaconsfield Buckinghamshire HP9 1RT (GB)**

LIBER, STOCKHOLM 1987

## Description

The present invention relates to a process for the manufacture of quinolinic acid.

Quinolinic acid is used as an intermediate for the synthesis of pharmaceuticals, insecticides, pigments and dyes. Additionally quinolinic acid is assuming increasing importance in the pressure sensitive record material art where it is needed as a starting material for the synthesis of chromogenic materials such as the chromogenic pyridinone materials disclosed in U.S. Patent No. 3775424.

Quinolinic acid has hitherto been manufactured by the oxidation of quinoline with hydrogen peroxide in the presence of copper sulphate. A recent development of this route is disclosed in European Patent No. 0024197. While this process represented a considerable advance in the art through the use of particular reaction conditions, the reaction itself is exothermic and in order to maintain control, it must be performed at a lower than optimum temperature, thus lowering the rate of reaction causing an increase in reaction time and an increase in the opportunity for the occurrence of side reactions.

A substantial improvement over the process of European Patent No. 0024197 is disclosed in European patent No. 0034943 by use of a combination of reduced pressure and reuse of the reaction medium. This latter process, nevertheless, requires the use of more complicated and expensive equipment and utilizes an oxidizing agent which can be decomposed, sometimes explosively, by the presence of certain contaminants such as rust.

A proposal for the laboratory synthesis of quinolinic acid was made by D.C. Ayres and A.M. M. Hossain in <u>J. Chem. Soc. perkin Trans. 1</u> 1975 (8) 707-10 (Chem. Abs. Vol. 83 1975 27980V) in which quinoline was oxidised with ruthenium tetraoxide in the presence of carbon tetrachloride and hypochlorite solution to form quinolinic acid which was isolated and characterised as the dimethyl ester. The oxidation step of this synthesis was not efficient since 60% of the quinoline was recovered unreacted. Of the remaining 40% of the quinoline, 45% was isolated as the dimethyl ester of quinolinic acid. This represents an overall yield of 18% based on the total amount of quinoline employed.

We have now found that unexpectedly high yields of quinolinic acid can be obtained when quinoline is oxidised with ruthenium tetraoxide in the presence of hypochlorite solution when the reaction medium contains a substantial molar excess of base over the quinoline starting material by the addition of base to the reaction medium beyond that provided to the reaction medium by the hypochlorite. Hypochlorite solution contains a certain amount of base as a consequence of the method used to manufacture the hypochloride, the residual base tending to act as a stabiliser for the hypochlorite solution. Additionally we have found that quinolinic acid of higher purity can be produced.

In a first aspect the preseut invention accordingly provides a process for the manufacture of quinolinic acid which comprises oxidising quinoline with ruthenium tetraoxide in the presence of an aqueous solution of hypochlorite characterised by the presence of sufficient additional base that the molar ratio of the total base to quinoline is at least 4:1.

In a second aspect the present invention provides a process for the manufacture of quinolinic acid which comprises oxidising quinoline with ruthenium tetraoxide In the presence of an aqueous solution of hypochlorite and sufficient sodium hydroxide and/or potassium hydroxide that the molar ratio of the total base to quinoline is at least 5:1.

In a third aspect the present invention provides a process for the manufacture of quinolinic acid which comprises oxidising quinoline with ruthenium tetraoxide in the presence of an aqueous solution of hypochlorite carbon tetrachloride and sufficient sodium carbonate and/or potassium carbonate that the molar ratio of the total base to quinoline is at least 4:1.

Chemically, in this process, the primary oxidant is ruthenium tetraoxide. In oxidising the quinoline the ruthenium tetraoxide is reduced to ruthenium dioxide which, in turn, is oxidized back to ruthenium tetraoxide by the secondary oxidant, the hypochlorite. The ruthenium tetraoxide can be provided to the reaction as such or by oxidation of a suitable source of ruthenium in effected <u>in situ</u> by the hypochlorite. The quantity of ruthenium used can be very small; we have successfully used amounts equivalent to about $5 \times 10^{-5}$ mol ruthenium per mol of quinoline. Larger amounts of the ruthenium catalyst may be utilised but above about 50 m mol. of the ruthenium catalyst per mol of quinoline the high cost of the catalyst becomes prohibitive.

The process of the present invention can be carried out by preparing a mixture of quinoline, a suitable source of ruthenium such as ruthenium trichloride, and base. While this mixture is stirred vigorously, hypochlorite solution is added and the stirring is continued until the reaction is complete. The quinolinic acid can be recovered from the reaction medium by precipitation as copper quinolinate. The copper quinolinate can be converted to quinolinic acid in a nearly quantitative manner by dispersing the copper quinolinate in water, heating to about 80°C and passing hydrogen sulphide gas through the dispersion.

Most water-soluble inorganic bases, are suitable for the performance of the present invention. Preferred strong bases are sodium hydroxide or potassium hydroxide and preferred weak bases are sodium or potassium carbonate. Generally the use of a strong base is preferred.

The amount of base which will be used depends upon the strength of the base. Typically for strong bases such as, for example, sodium hydroxide and potassium hydroxide, sufficient base will be added to the reaction medium to bring the molar ratio of the total base to quinoline to about 5 or greater. The upper limit is decided more by economic considerations than any additional beneficial effect on the reaction. A molar ratio of base: quinoline as high as 24:1 when sodium hydroxide was employed as the base has been found to provide unexpectedly enhanced yields. For strong bases a base:quinoline molar ratio of about 7:1 to about 12:1 is especially desired. For weaker bases such as, for example, sodium carbonate or potassium carbonate, the total base to quinoline molar ratio is 4:1 or greater, for example about 5:1 to about 12:1.

When a weak base is used, the reaction mixture used in the present invention will in practice include carbon tetrachloride to achieve the desired high yields. Carbon tetrachloride can, optionally and desirably be included when a strong base is employed but its use is not essential to obtain high yields. The hypochlorite solution can be any common metallic salt of hypochlorous acid but sodium hypochlorite in the weight percent range of about 5 to 15% is desirably used because it is readily available. Potassium hypochlorite is a suitable alternative. The theoretical amount of hypochlorite solution required for the oxidation is about 8 moles per mole of quinoline. To compensate for decomposition of the hypochlorite at elevated temperatures additional hypochlorite beyond the theoretical amount will be employed. We have obtained satisfactory results using an overall molar ratio of hypochlorite to quinoline of from 10:1 to 12:1. This additional amount can be readily determined experimentally.

The oxidation reaction is desirably performed at ambient or moderately superambient temperatures of from about 20°C to about 60°C and especially from about 40°C to about 55°C.

The yield of quinolinic acid does not vary much with increased temperature, however increasing the reaction temperature gives the advantage of shorter reaction times.

The process will usually be run for sufficient time to permit completion of the reaction. Twenty hours is usually sufficient but longer periods of time do not appear to adversely affect the yields.

The following examples illustrate the invention. All parts and percentages are by weight, unless specified otherwise. All solutions, unless otherwise designated, are aqueous solutions.

**Examples 1-13**

Examples 1-13 illustrate the preparation of quinolinic acid according to the present invention in the presence of a strong base.

The following amounts, volumes and reaction conditions were utilized in each of the 13 experiments:

| | |
|---|---|
| amount of quinoline | 5.2 g; 0.04 mol |
| amount of sodium hypochlorite | 0.44 mol |
| amount of ruthenium trichloride | 0.01 g; $3.8 \times 10^{-5}$ mol |
| volume of carbon tetrachloride | 20 $cm^3$(ml) |
| volume of aqueous phase | 350 $cm^3$(ml) |
| reaction temperature | 50°C |
| reaction time | 20 hours |

Prior to each experiment, the hypochlorite solution employed was analysed for the amount of base present and the concentration of the hypochlorite. The amount of base found in the hypochlorite solutions employed typically contributed to the base to quinoline ratio in the range of from 0.625:1 to 1.5:1.

For each experiment, a mixture of 5.2 g of quinoline, 0.01 g of ruthenium trichloride trihydrate and sufficient sodium hydroxide to provide the desired total base to quinoline molar ratio was stirred vigorously and sufficient sodium hypochlorite solution to provide 0.44 mol of sodium hypochlorite was added to the stirring mixture. The sodium hydroxide was added in a volume of water determined by the difference between 350 $cm^3$(ml) and the volume of sodium hypochlorite solution utilised. The stirring mixture was heated to 50°C and the stirring and heating was maintained under reflux conditions for 20 hours.

The quinolinic acid product from each reaction was recovered from the cooled reaction mixture by the following procedure. The aqueous layer was separated from the reaction mixture. 10 $cm^3$(ml) of isopropyl alcohol were added and the resulting liquid was filtered to remove insoluble material. The filtrate was acidified to pH 1.0 with dilute sulphuric acid, 20 g (0.08 mol) of copper sulphate pentahydrate in 30 $cm^3$(ml) water were added, the mixture was heated to 80°C for 30 minutes and the precipitated copper quinolinate was removed by filtration and dried in an oven at 100°C for 2 to 3 hours.

Using the above procedure Examples 1-13 were prepared. The molar ratio of total base (the amount found by analysis in the hypochlorite solution plus the amount added) to quinoline and the corresponding yield of copper quinolinate (based on the quinoline used) are entered in Table 1 for Examples 1-13.

**Table 1**

| Example | Molar ratio of total base to quinoline | Yield of copper quinolinate (%) |
|---|---|---|
| 1 | 1 | 0 |
| 2 | 2 | trace |
| 3 | 3 | 21 |
| 4 | 4 | 46 |
| 5 | 5 | 74 |
| 6 | 6 | 76 |
| 7 | 7 | 83 |
| 8 | 8 | 81 |
| 9 | 9 | 92 |
| 10 | 10 | 82 |
| 11 | 12 | 92 |
| 12 | 14 | 75 |
| 13 | 24 | 73 |

**Examples 14-22**

Examples 14-22 were performed using the same procedure as was used for Examples 1-13 except that the added base was sodium carbonate rather than sodium hydroxide. Listed in Table 2 are the molar ratio of total base to quinoline and yield of copper quinolinate for each Example.

**Table 2**

| Example | molar ratio of total base to quinoline | Yield of copper quinolinate (%) |
|---|---|---|
| 14 | 2 | 35 |
| 15 | 3 | 43 |
| 16 | 4 | 51 |
| 17 | 5 | 51 |
| 18 | 6 | 54 |
| 19 | 7 | 59 |
| 20 | 8 | 54 |
| 21 | 9 | 54 |
| 22 | 10 | 60 |

4

**Example 23**

A mixture of 5.2 g (0.04 mol) of quinoline, 0.01 g (3.8 x 10$_{-5}$ mol) ruthenium trichloride trihydrate, 20 cm$^3$(ml) of carbon tetrachloride and 12.3 g (0.22 mol) of potassium hydroxide in 20 cm$^3$(ml) of water was stirred vigorously and 260 cm$^3$(ml) of 12.7% sodium hypochlorite solution (0.44 mol hypochlorite) was added to the stirring mixture. The molar ratio of total base to quinocline was about 6.5. The stirring mixture was heated to 50°C and refluxed at 50°C with stirring for 20 hours.

The quinolinic acid product from the above reaction was recovered from the reaction mixture by the same procedure as in Examples 1-13. The yield of quinolinic acid in the form of copper quinolinate was 7.8 g (74%).

**Examples 24 to 29**

The general method of Example 1 was repeated using 12.5 mol. sodium hypochlorite and about 6 mol total base (NaOH) at a reaction temperature of about 30°C but using differing amounts of ruthenium trichloride. The results are set out in Table 4 below.

**Examples 30 to 44**

Example 24 was repeated but the carbon tetrachloride was omitted and varying quantities of sodium hypochlorite, base (NaOH), reaction temperature and ruthenium trichloride were used to see how the yield varied with these changes. The results are set out in Table 4 below.

it will be noted that high yields were maintained. The effect of reaction temperature on yield is not marked but the use of higher reaction tempertures may permit shorter reaction times thus the reaction at 40°C going to completion after about 20 hrs was very nearly complete after 7 hrs at 55°C.

**Table 4**

| EXAMPLE | MOLAR RATIO QUINOLINE TO: | | | TEMP | YIELD |
| NO | OCl | Ru | Base | °C | % |
| | | x1000 | | | |
| --- | --- | --- | --- | --- | --- |
| 24 | 12.5 | 20 | 6 | 25-30 | 89.8 |
| 25 | 12.5 | 10 | 6 | 25-30 | 85.9 |
| 26 | 12.5 | 5 | 6 | 25-30 | 87.8 |
| 27 | 12.5 | 2 | 6 | 25-30 | 82.9 |
| 28 | 12.5 | 1 | 6 | 25-30 | 84.9 |
| 29 | 12.5 | 0.2 | 6 | 25-30 | 59.5 |
| 30 | 10 | 0.1 | 7 | 35 | 82.0 |
| 31 | 11 | 0.1 | 7 | 35 | 82.2 |
| 32 | 12 | 0.1 | 7 | 35 | 76 |
| 33 | 10 | 0.1 | 5 | 55 | 82.2 |
| 34 | 10 | 0.1 | 7 | 55 | 89.8 |
| 35 | 10 | 0.1 | 8.5 | 55 | 87.8 |
| 36 | 11 | 0.1 | 7 | 50 | 91.2 |
| 37 | 11 | 0.1 | 7 | 55 | 92.2 |
| 38 | 11 | 0.1 | 7 | 60 | 90.5 |
| 39 | 11.5 | 0.5 | 7 | 30 | 81 |
| 40 | 11.5 | 0.25 | 7 | 30 | 81 |
| 41 | 11.5 | 0.2 | 7 | 30 | 80 |
| 42 | 11.5 | 0.15 | 7 | 30 | 81.5 |
| 43 | 11.5 | 0.1 | 7 | 30 | 72.7 |
| 44 | 11.5 | 0.05 | 7 | 30 | 67.3 |

**Claims**

1. A process for the manufacture of quinolinic acid which comprises oxidising quinoline with ruthenium tetraoxide in the presence of an aqueous solution of hypochlorite characterised by the presence of sufficient additional base that the molar ratio of the total base to quinoline is at least 4:1.

2. A process as claimed in Claim 1 wherein the hypochlorite solution is sodium hypochlorite and/or potassium hypochlorite.

3. A process as claimed in either Claim 1 or Claim 2 wherein the base is sodium hydroxide and/or potassium hydroxide.

4. A process as claimed in either Claim 1 or Claim 2 wherein the base is sodium carbonate and/or potassium carbonate.

5. A process as claimed in any preceding claim wherein the molar ratio of the total base to quinoline is at least 5:1.

6. A process as claimed in Claim 5 wherein the molar ratio of the total base to quinoline is from 7:1 to 12:1.

7. A process as claimed in any preceding claim wherein the reaction is carried out in the presence of carbon tetrachloride.

8. A process as claimed in any preceding claim wherein the oxidation is conducted at a temperature from 20°C to 60°C.

9. A process as claimed in Claim 8 wherein the oxidation is conducted at a temperature from 40°C to 55°C.

10. A process for the manufacture of quinolinic acid which comprises oxidising quinoline with ruthenium tetraoxide in the presence of an aqueous solution of hypochlorite and sufficient sodium hydroxide and/or potassium hydroxide that the molar ratio of the total base to quinoline is at least 5:1.

11. A process for the manufacture of quinolinic acid which comprises oxidising quinoline with ruthenium tetraoxide in the presence of an aqueous solution of hypochlorite, carbon tetrachloride and sufficient sodium carbonate and/or potassium carbonate that the molar ratio of the total base to quinoline is at least 4:1.


**Patentansprüche**

1. Verfahren zur Herstellung von Chinolinsäure durch Oxidation von Chinolin mit Rutheniumtetraoxid in Gegenwart einer wäßrigen Lösung von Hypochlorit, gekennzeichnet durch die Gegenwart von ausreichend zusätzlicher Base, damit das Molverhältnis der gesamten Base zum Chinolin mindestens 4:1 beträgt.

2. Verfahren nach Anspruch 1, dadurch gekennzeichnet, daß die Hypochlorit-Lösung Natriumhypochlorit und/oder Kaliumhypochlorit ist.

3. Verfahren nach Anspruch 1 oder 2, dadurch gekennzeichnet, daß die Base Natriumhydroxid und/oder Kaliumhydroxid ist.

4. Verfahren nach Anspruch 1 oder 2, dadurch gekennzeichnet, daß die Base Natriumcarbonat und/oder Kaliumcarbonat ist.

5. Verfahren nach einem der vorhergehenden Ansprüche, dadurch gekennzeichnet, daß das Molverhältnis der gesamten Base zum Chinolin mindestens 5:1 ist.

6. Verfahren nach Anspruch 5, dadurch gekennzeichnet, daß das Molverhältnis der gesamten Base zum Chinolin 7:1 bis 12:1 beträgt. 7. Verfahren nach einem der vorhergehenden Ansprüche, dadurch gekennzeichnet, daß die Reaktion in Gegenwart von Kohlenstofftetrachlorid durchgeführt wird.

8. Verfahren nach einem der vorhergehenden Ansprüche, dadurch gekennzeichnet, daß die Oxidation bei einer Temperatur von 20°C bis 60°C durchgeführt wird.

9. Verfahren nach Anspruch 8, dadurch gekennzeichnet, daß die Oxidation bei einer Temperatur von 40°C bis 55°C durchgeführt wird.

10. Verfahren zur Herstellung von Chinolinsäure durch Oxidation von Chinolin mit Rutheniumtetraoxid in Gegenwart einer wäßrigen Lösung von Hypochlorit und ausreichend Natriumhydroxid und/oder Kaliumhydroxid, damit das Molverhältnis der gesamten Base zum Chinolin mindestens 5:1 beträgt.

11. Verfahren zur Herstellung von Chinolinsäure durch Oxidation von Chinolin mit Rutheniumtetraoxid in Gegenwart einer wäßrigen Lösung von Hypochlorit, Kohlenstofftetrachlorid und ausreichend Natriumcarbonat und/oder Kaliumcarbonat, damit das Molverhältnis der gesamten Base zum Chinolin mindestens 4:1 beträgt.


**Revendications**

1. Procédé de préparation de l'acide quinoléique par l'oxydation de la quinoléine par le tétroxyde de ruthénium en présence d'une solution aqueuse d'un hypochlorite, caractérisé en ce que le milieu réactionnel contient une base additionnelle en une proportion telle que le rapport molaire base totale - quinoléine est au moins égal à 4:1.

2. Procédé suivant la revendication 1, dans lequel la solution aqueuse d'hypochlorite est une solution d'hypochlorite de sodium et (ou) d'hypochlorite de potassium.

3. Procédé suivant la revendication 1 ou la revendication 2, dans lequel la base est de l'hydroxyde de sodium et(ou) de l'hydroxyde de potassium.

4. Procédé suivant la revendication 1 ou la revendication 2, dans lequel la base est du carbonate de sodium et ou) du carbonate de potassium.

5. Procédé suivant l'une quelconque des revendications précédentes, dans lequel le rapport molaire base totale - quinoléine est au moins égal à 5:1.

6. Procédé suivant la revendication 5, dans lequel le rapport molaire base totale - quinoléine est compris entre 7:1 et 12:1.

7. Procédé suivant l'une quelconque des revendications précédentes, dans lequel la réaction est effectuée en présence de tétrachlorure de carbone.

8. Procédé suivant l'une quelconque des revendications précédentes, dans lequel l'oxydation est réalisée à une température comprise entre 20 et 60°C.

9. Procédé suivant la revendication 8, dans lequel l'oxydation est réalisée entre 40 et 55°C.

10. Procédé de préparation de l'acide quinoléique, consistant à oxyder la quinoléine par le tétroxyde de ruthénium en présence d'une solution aqueuse d'hypochlorite et d'une proportion suffisante d'hydroxyde de sodium et(ou) d'hydroxyde de potassium pour obtenir un rapport molaire base totale - quinoléine au moins égal à 5: 1.

11. Procédé de préparation de l'acide quinoléique, consistant à oxyder la quinoléine par le tétroxyde de ruthénium en présence d'une solution aqueuse d'hypochlorite, de tétaschlorure de carbone et d'une proportion suffisante de carbonate de sodium et(ou) de carbonate de potassium pour obtenir un rapport molaire base totale - quinoléine au moins égal à 4:1.